(19) Europäisches Patentamt
European Patent Office
Office européen des brevets

(11) **EP 3 363 363 B1**

(12) **EUROPÄISCHE PATENTSCHRIFT**

(45) Veröffentlichungstag und Bekanntmachung des Hinweises auf die Patenterteilung:
**17.11.2021   Patentblatt 2021/46**

(51) Int Cl.:
*A61B 6/03* *(2006.01)*      *A61B 6/00* *(2006.01)*

(21) Anmeldenummer: **17178749.2**

(22) Anmeldetag: **29.06.2017**

(54) **VERFAHREN ZUM DURCHFÜHREN EINER BILDGEBENDEN UNTERSUCHUNG**

METHOD FOR PERFORMING AN IMAGING EXAMINATION

PROCÉDÉ D'EXÉCUTION D'UN EXAMEN POUR L'IMAGERIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**22.08.2018   Patentblatt 2018/34**

(73) Patentinhaber: **Siemens Healthcare GmbH**
**91052 Erlangen (DE)**

(72) Erfinder: **Hofmann, Christian**
**91052 Erlangen (DE)**

(56) Entgegenhaltungen:
**EP-A2- 0 370 341      US-A1- 2011 286 574**
**US-A1- 2012 014 499**

EP 3 363 363 B1

**Beschreibung**

Die Erfindung betrifft ein Verfahren zum Durchführen einer bildgebenden Untersuchung eines Patienten mittels eines Computertomographen, den zugehörigen Computertomographen und ein zugehöriges Computerprogrammprodukt.

Für eine Bestrahlungsplanung eines Patienten beispielsweise mit einem Lungen- oder Abdomen-Karzinom werden üblicherweise drei-dimensionale 3D Bilder eines Messbereichs mit einer Ausdehnung in z-Richtung verwendet. Mittels eines Computertomographen können insbesondere Projektionsdaten bei einer bildgebenden Untersuchung erfasst werden, von welchen die 3D-Bilder rekonstruiert werden können. Die bildgebende Untersuchung mittels des Computertomographen erfordert üblicherweise ionisierende Röntgenstrahlen. Die 3D-Bilder umfassen insbesondere mehrere Schichtbilder des Patienten. Um eine für die Bestrahlungsplanung relevante Anatomie, insbesondere das Lungen- oder Abdomen-Karzinom, des Patienten in einer bestimmten Atemphase des Patienten rekonstruieren zu können, wird während der bildgebenden Untersuchung eine Atembewegung des Patienten in Echtzeit erfasst. Vorzugsweise werden für jede z-Position des Messbereichs die Schichtbilder über alle Atemphasen eines Atemzyklus, welcher insbesondere ein Einatmen und Ausatmen des Patienten abbildet und einem periodischen Segment der Atembewegung entspricht, rekonstruiert. Damit werden 4D-Bilder bzw. atemkorrelierte 3D-Bilder-Serien generiert. Dadurch kann insbesondere eine besonders geeignete Anpassung der Dosisverteilung an ein sich in Bewegung befindendes Planungs-Zielvolumen sichergestellt werden.

Die Bestrahlungsplanung hängt kritisch von einer Bildqualität der 4D-Bilder ab. Für die Bildqualität sind in erster Linie Messparameter der bildgebenden Untersuchung relevant, wobei die Messparameter vorzugsweise optimal berechnet werden. Typischerweise werden die Messparameter bisher aus einer Liste mit, insbesondere für einen Nutzer, unveränderlichen Messparametern für die bildgebende Untersuchung gewählt. Die unveränderlichen Messparameter sind üblicherweise nicht in Bezug auf die Bildqualität optimiert. Die Nachteile, die sich daraus ergeben, sind, dass durch einen Bedienfehler des Nutzers ein unpassender Satz an Messparametern gewählt wird.

Die US 2011/0286574 A1 offenbart ein Verfahren zur Durchführung einer bildgebenden Untersuchung an mehreren z-Positionen in Abhängigkeit von einer periodischen Bewegung.

Die EP 0 370 341 A2 offenbart ein Verfahren zur Reduktion von Bildartefakten aufgrund einer periodischen Bewegung bei einer bildgebenden Untersuchung.

Der Erfindung liegt die Aufgabe zu Grunde, ein auf den Patienten individuell abgestimmtes Verfahren zum Durchführen einer bildgebenden Untersuchung eines Patienten mittels eines Computertomographen, den zugehörigen Computertomographen und ein zugehöriges Computerprogrammprodukt anzugeben.

Die Aufgabe wird durch die Merkmale des unabhängigen Anspruchs gelöst. Vorteilhafte Weiterbildungen sind in den abhängigen Ansprüchen angegeben.

Nachstehend wird die erfindungsgemäße Lösung der Aufgabe sowohl in Bezug auf den beanspruchten Computertomographen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche die beispielsweise auf den Computertomographen gerichtet sind auch mit den Merkmalen, die in Zusammenhang mit dem Verfahren beschrieben oder beansprucht sind, weitergebildet sein. Die entsprechenden funktionalen Merkmale des Verfahrens werden dabei durch entsprechende gegenständliche Module ausgebildet.

Das erfindungsgemäße Verfahren zum Durchführen einer bildgebenden Untersuchung eines Patienten mittels eines Computertomographen umfasst die Schritte:

- Erfassen einer Atembewegung des Patienten, wobei von der Atembewegung des Patienten ein atemkorrelierter Parameter $T_{cycle}$ ermittelt wird, welcher eine Zeitdauer eines Atemzyklus der Atembewegung beschreibt,
- Festlegen eines Messbereichs der bildgebenden Untersuchung, wobei der Messbereich zumindest eine z-Position aufweist,
- automatisches Berechnen zumindest eines Messparameters gemäß der Atembewegung, wobei der atemkorrelierte Parameter $T_{cycle}$ als Eingangsparameter für das automatische Berechnen des zumindest einen Messparameters derart verwendet wird, dass bei einem Durchführen der bildgebenden Untersuchung gemäß dem zumindest einen Messparameter Projektionsdaten an der zumindest einen z-Position über die gesamte Zeitdauer des Atemzyklus erfasst werden, und
- Durchführen der bildgebenden Untersuchung des Patienten gemäß dem zumindest einen Messparameter in dem Messbereich mittels des Computertomographen, wobei die Projektionsdaten akquiriert werden, welche an der zumindest einen z-Position den Atemzyklus des Patienten über die gesamte Zeitdauer des Atemzyklus abbilden.

Das Erfassen der Atembewegung des Patienten erfolgt insbesondere mittels eines Sensors, beispielsweise mittels einer Kamera oder eines Atemgurts. Die Atembewegung beschreibt üblicherweise ein freies Atmen des Patienten und umfasst insbesondere wenigstens einen Atemzyklus des Patienten. Die Atembewegung wird insbesondere über den gesamten Atemzyklus des Patienten erfasst, wobei der gesamte Atemzyklus zumindest ein einmaliges Einatmen und einmaliges Ausatmen des Patienten umfasst. Der Atemzyklus entspricht vorzugsweise einem periodischen Segment der Atembewegung. Die Zeitdauer des periodischen Segments entspricht der Zeitdauer des Atemzyklus. Das Ermitteln des atemkorrelierten Parameter $T_{cycle}$ erfolgt insbesondere automatisch gemäß einem geeigneten Algorithmus. Je langsamer der Patient atmet, desto höher ist insbesondere der atemkorrelierte Parameter $T_{cycle}$. Vorzugsweise entspricht der atemkorrelierte Parameter der Zeitdauer des Atemzyklus.

Das Festlegen des Messbereichs der bildgebenden Untersuchung kann durch einen Nutzer des Computertomographen erfolgen. Der Computertomograph weist dafür eine Planungseinheit auf, welche einen Monitor und zumindest ein Eingabegerät aufweist. Der Nutzer legt den Messbereich insbesondere mittels des zumindest einen Eingabegeräts auf einer graphischen Benutzeroberfläche des Monitors fest. Beispielsweise werden dem Nutzer auf der graphischen Benutzeroberfläche Übersichtsbilder, die von bei einer Übersichtsmessung erfassten Übersichtsdaten rekonstruiert werden, angezeigt. Grundsätzlich ist denkbar, dass der Messbereich der bildgebenden Untersuchung automatisch, insbesondere mittels sensorgestützten Erfassen des Patienten und/oder mittels Landmarkenerkennung, festgelegt wird.

Der Messbereich der bildgebenden Untersuchung ist insbesondere in einer sich in Längsrichtung des Patienten erstreckenden Längsachse ausgedehnt. Die Längsachse des Patienten ist vorzugsweise parallel zu einer sich in Längsrichtung einer Patientenliege erstreckenden Längsachse. Üblicherweise entspricht eine z-Richtung des Computertomographen der Längsachse der Patientenliege. Der Messbereich weist die zumindest eine z-Position auf, wobei die zumindest eine z-Position eine Position einer Schicht entlang der Längsachse der Patientenliege beschreibt. Die Schicht entlang der Längsachse der Patientenliege wird insbesondere von einem Schichtbild abgebildet.

Das Schichtbild weist insbesondere eine Schichtdicke mit Ausdehnung in z-Richtung des Computertomographen auf. Der Messbereich umfasst vorzugsweise ein 3D-Volumen mit einer Ausdehnung in z-Richtung des Computertomographen. In diesem Fall kann entweder ein einzelnes Schichtbild mit einer hohen Schichtdicke oder mehrere Schichtbilder mit einer niedrigen Schichtdicke rekonstruiert werden. Üblicherweise werden desto mehr Schichtbilder rekonstruiert, je niedriger die Schichtdicke der jeweiligen Schichtbilder ist.

Bei der bildgebenden Untersuchung können wenigstens drei Parametergruppen unterschieden werden. Die erste Parametergruppe umfasst insbesondere fixe Parameter, welche beispielsweise durch den Patienten oder durch eine technische Ausführung des Computertomographen vorgegeben sind. Beispiele für fixe Parameter sind ein Alter des Patienten oder eine Röhren-Detektor-Geschwindigkeit, welche die Geschwindigkeit beschreibt, mit welcher sich zumindest ein Röntgenstrahler des Computertomographen und zumindest ein Röntgendetektor des Computertomographen um den Patienten rotieren können. Insbesondere der atemkorrelierte Parameter $T_{cycle}$ ist der ersten Parametergruppe zugeordnet.

Die zweite Parametergruppe umfasst modifizierbare Parameter, welche insbesondere durch den Nutzer des Computertomographen beispielsweise auf der graphischen Benutzeroberfläche festgelegt werden können. Beispiele für modifizierbare Parameter sind eine Gesamtdosis $I_{ges}$, welche direkt proportional zu einer weiteren Messgröße einer Strahlenbelastung CTDI (engl. Computed Tomography Dose Index) des Patienten bei der bildgebenden Untersuchung ist, und ein Rekonstruktionskernel für eine Rekonstruktion eines Schichtbildes von den Projektionsdaten. Die Gesamtdosis $I_{ges}$ gibt insbesondere die Bildqualität des Schichtbildes sowie die Strahlenbelastung des Patienten bei dem Durchführen der bildgebenden Untersuchung an.

Die dritte Parametergruppe weist abhängige Parameter auf, welche beispielsweise von Parametern aus der ersten Parametergruppe und/oder aus der zweiten Parametergruppe berechnet werden können. Beispielsweise kann ein Rekonstruktionswinkelintervall theta berechnet werden. Üblicherweise kann der zumindest eine Messparameter der dritten Parametergruppe zugeordnet werden. Das automatische Berechnen des zumindest einen Messparameters gemäß der Atembewegung beschreibt insbesondere das automatische Berechnen eines abhängigen Parameters aus der dritten Parametergruppe gemäß einem fixen Parameter aus der ersten Parametergruppe, insbesondere gemäß dem atemkorrelierten Parameter $T_{cycle}$. Der atemkorrelierte Parameter $T_{cycle}$ und ein modifizierbarer Parameter der zweiten Parametergruppe gehen insbesondere derart als die Eingangsparameter für das automatische Berechnen ein, dass bei dem Durchführen der bildgebenden Untersuchung die Projektionsdaten an der zumindest einen z-Position über die gesamte Zeitdauer des Atemzyklus akquiriert werden können. In anderen Worten wird der zumindest eine Messparameter derart automatisch berechnet, dass für jeden Zeitpunkt des Atemzyklus die Projektionsdaten an der zumindest einen z-Position erfasst werden. Mittels der Projektionsdaten an der zumindest einen z-Position können daher das Einatmen und das Ausatmen des Patienten an der zumindest einen z-Position während der bildgebenden Untersuchung abgebildet werden. Wenn die Projektionsdaten an der zumindest einen z-Position den Atemzyklus des Patienten über die gesamte Zeitdauer des Atemzyklus abbilden, wird üblicherweise von einer atemkorrelierten bildgebenden Untersuchung gesprochen.

Das automatische Berechnen weist insbesondere zumindest eine Formel auf, wobei der zumindest eine Messparameter gemäß der zumindest einen Formel derart automatisch berechnet wird, dass bei dem Durchführen der bildgebenden Untersuchung die Projektionsdaten an der zumindest einen z-Position den Atemzyklus des Patienten über die gesamte Zeitdauer des Atemzyklus abbilden können. Der atemkorrelierte Parameter $T_{cycle}$ stellt dann insbesondere ein Rechenglied in der Formel dar, mittels welcher der zumindest eine Messparameter automatisch berechnet wird.

Das automatische Berechnen erfolgt insbesondere in einer Recheneinheit des Computertomographen. Die Recheneinheit weist dafür vorteilhafterweise einen Speicher auf, in welchen Programmcodemittel ladbar sind. Die Programmcodemittel weisen insbesondere die zumindest eine Formel zum automatischen Berechnen des zumindest einen Messparameters auf.

Das automatische Berechnen ist besonders vorteilhaft, weil der zumindest eine Messparameter für jeden Patienten individuell automatisch berechnet wird und damit die bildgebende Untersuchung besonders geeignet an den Patienten angepasst wird, weil der zumindest eine Messparameter nicht aus über ein Patientenkollektiv gemittelte vorgegebene Parametersätze ausgewählt wird.

Bei dem Durchführen der bildgebenden Untersuchung mit einem Pitch wird die Patientenliege kontinuierlich durch ein maximales Gesichtsfeld des Computertomographen hindurch geschoben. Dieses Vorgehen wird normalerweise spirale Akquisition von Projektionsdaten genannt. Typische Werte eines Pitch bei einer Durchführung einer Messung in dem Computertomographen liegen über 0 und unter 2. Alternativ zu der spiralen Akquisition kann die Patientenliege in diskreten Schritten durch das maximale Gesichtsfeld des Computertomographen hindurch geschoben werden. In diesem Fall werden nach jedem diskreten Schritt vorzugsweise für die zumindest eine z-Position die Projektionsdaten über die gesamte Zeitdauer des Atemzyklus erfasst.

Der Pitch ist proportional zu einem Tischvorschub der Patientenliege und einer Ausdehnung des Röntgendetektors in z-Richtung des Computertomographen. Das maximale Gesichtsfeld hängt insbesondere von der Ausdehnung ab. Die bildgebende Untersuchung wird vorzugsweise mit einem kleinen Pitch in der Größenordnung von 0,1 bei einer minimalen Röhrenrotationumlaufzeit $T_{rot}$ des Computertomographen von 0,5s durchgeführt.

Der Pitch entspricht insbesondere einem Verhältnis der minimalen Röhrenrotationumlaufzeit $T_{rot}$ zu dem atemkorrelierten Parameter $T_{cycle}$. Die minimale Röhrenrotationumlaufzeit $T_{rot}$ ist üblicherweise spezifisch für jeden Computertomographen durch die technische Ausführung festgelegt und kann nicht verändert werden. Je niedriger die minimale Röhrenrotationumlaufzeit $T_{rot}$ ist, desto höher ist die Röhren-Detektor-Geschwindigkeit, desto schneller rotieren insbesondere der Röntgenstrahler und der Röntgendetektor um den Patienten herum und/oder desto mehr Projektionsdaten werden pro Zeiteinheit erfasst. Üblicherweise rotieren insbesondere der zumindest eine Röntgenstrahler und der zumindest eine Röntgendetektor fortwährend während der bildgebenden Untersuchung.

Wenn die Patientenliege durch das maximale Gesichtsfeld geschoben wird, wird üblicherweise während der Akquisition der Projektionsdaten ein Winkel, in welchem sich der zumindest eine Röntgenstrahler und der zumindest eine Röntgendetektor relativ zur Patientenliege befinden, und eine aktuelle z-Position der Patientenliege beispielsweise mit Bezug auf die Projektionsdaten gespeichert.

Das Durchführen der bildgebenden Untersuchung erfolgt insbesondere, nachdem der zumindest eine Messparameter vorzugsweise patientenindividuell, insbesondere gemäß der Atembewegung des Patienten, automatisch berechnet worden ist. Die bildgebende Untersuchung kann gemäß dem vorgeschlagenen Vorgehen insbesondere daher vorteilhaft durchgeführt werden, weil die die Projektionsdaten den Atemzyklus des Patienten über die gesamte Zeitdauer des Atemzyklus abbilden können. Derart können insbesondere Artefakte durch die Atembewegung des Patienten unterdrückt werden. Außerdem kann durch das automatische Berechnen die bildgebenden Untersuchung dahingehend optimiert werden, dass der Patient nicht länger als nötig für das Erfassen der Projektionsdaten an der zumindest einen z-Position über die gesamte Zeitdauer des Atemzyklus in dem Computertomographen mit den ionisierenden Röntgenstrahlen bestrahlt wird.

Für das automatische Berechnen des zumindest einen Messparameters ist insbesondere keine Benutzerinteraktion nötig. Dadurch kann ein fehlerhaftes Festlegen des zumindest einen Messparameters verhindert werden.

Die Projektionsdaten bilden an der zumindest einen z-Position den Atemzyklus des Patienten über die gesamte Zeitdauer des Atemzyklus ab. Typischerweise werden von den akquirierten Projektionsdaten Bilddaten, insbesondere das Schichtbild, rekonstruiert und in einer Datenbank abgespeichert und/oder auf dem Monitor zu einer Befundung durch einen Arzt bereitgestellt. Vorteilhafterweise wird das rekonstruierte Schichtbild als Grundlage für eine Erstellung eines Strahlentherapieplans verwendet.

Eine Ausführungsform sieht vor, dass der atemkorrelierte Parameter $T_{cycle}$ derart ermittelt wird, dass die Atembewegung in periodische Segmente unterteilt wird und die Zeitdauer des Atemzyklus gemäß einem Median von Zeitdauern der periodischen Segmente berechnet wird. Vorzugsweise wird durch das Unterteilen der Atembewegung eine Stichprobe von periodischen Segmenten generiert, wobei insbesondere für jedes periodische Segment die Zeitdauer berechnet und dann der Median über die Zeitdauern gebildet wird. Der atemkorrelierte Parameter $T_{cycle}$ kann insbesondere auf Grundlage der so berechneten Zeitdauer ermittelt werden. Typischerweise wird der Median über die Zeitdauern, insbesondere die so berechnete Zeitdauer, als atemkorrelierter Parameter $T_{cycle}$ gesetzt.

Eine Ausführungsform sieht vor, dass der atemkorrelierte Parameter $T_{cycle}$ gemäß einer Standardabweichung von den Zeitdauern der periodischen Segmente angepasst wird. Aus der Stichprobe von den periodischen Segmenten kann die Standardabweichung der Zeitdauern der periodischen Segmente berechnet werden. Die Standardabweichung gibt insbesondere ein Maß für eine Irregularität der Zeitdauern der periodischen Segmente und damit das Maß für die Irregularität der Atembewegung des Patienten an. Der atemkorrelierte Parameter $T_{cycle}$ wird insbesondere mit einem desto größeren Faktor multipliziert, je höher die Standardabweichung ist. Der Faktor ist dabei insbesondere stets größer als eins.

Das automatische Berechnen des zumindest einen Messparameters umfasst eine derartige Berechnung eines Röhrenstroms $I_{tube}$ des Computertomographen bei der bildgebenden Untersuchung, dass der Röhrenstrom $I_{tube}$ indirekt proportional zum atemkorrelierten Parameter $T_{cycle}$ ist. Je höher der atemkorrelierte Parameter $T_{cycle}$ ist, desto niedriger ist insbesondere der Röhrenstrom $I_{tube}$. Daraus folgt, dass der Röhrenstrom $I_{tube}$ vorzugsweise desto niedriger ist, je langsamer der Patient atmet. Wenn der Röhrenstrom $I_{tube}$ indirekt proportional zum atemkorrelierten Parameter $T_{cycle}$ ist, wird insbesondere die Gesamtdosis $I_{ges}$ konstant gehalten. Durch das derartige automatische Berechnen des zumindest einen Messparameters wird vorteilhafterweise die Gesamtdosis $I_{ges}$ der bildgebenden Untersuchung nicht verändert.

Eine Ausführungsform sieht vor, dass das automatische Berechnen des zumindest einen Messparameters eine derartige Berechnung eines schicht-effektiven Röhrenstrom-Zeit-Produkts $I_{rot}$ umfasst, dass das schicht-effektive Röhrenstrom-Zeit-Produkt $I_{rot}$ einem Produkt aus dem Röhrenstrom $I_{tube}$ des Computertomographen bei der bildgebenden Untersuchung und aus der minimalen Röhrenrotationumlaufzeit $T_{rot}$ des Computertomographen bei der bildgebenden Untersuchung entspricht. Je höher der Röhrenstrom $I_{tube}$ ist, desto höher ist insbesondere das schicht-effektive Röhrenstrom-Zeit-Produkt $I_{rot}$. Gleichermaßen ist das schicht-effektive Röhrenstrom-Zeit-Produkt $I_{rot}$ desto höher, je höher die minimale Röhrenrotationumlaufzeit $T_{rot}$ des Computertomographen bei der bildgebenden Untersuchung ist. Das schicht-effektive Röhrenstrom-Zeit-Produkt $I_{rot}$ gibt insbesondere ein Maß einer emittierten Röntgenstrahlmenge an und ist typischerweise proportional zu der Strahlenbelastung. Je höher das schicht-effektive Röhrenstrom-Zeit-Produkt $I_{rot}$ ist, desto höher ist üblicherweise die Strahlenbelastung.

In die Berechnung des schicht-effektiven Röhrenstrom-Zeit-Produkts $I_{rot}$ geht insbesondere der Röhrenstrom $I_{tube}$, welcher indirekt proportional von dem atemkorrelierten Parameter $T_{cyc-le}$ abhängt, ein.

Das Rekonstruktionswinkelintervall theta gibt insbesondere ein Winkelintervall für die Rekonstruktion des Schichtbildes an. Das Rekonstruktionswinkelintervall theta umfasst insbesondere einen Winkelbereich von mindestens 180°, was üblicherweise für die Rekonstruktion des Schichtbildes ein Minimum darstellt. Je größer das Rekonstruktionswinkelintervall theta ist, desto mehr Projektionsdaten mit dem zumindest einen Winkel innerhalb des Rekonstruktionswinkelintervalls theta werden bei der Rekonstruktion des Schichtbildes verwendet. Wenn das Rekonstruktionswinkelintervall theta mindestens 180° beträgt, können üblicherweise derartige Bildartefakte im Schichtbild minimiert werden, welche insbesondere durch ein Rekonstruktionswinkelintervall unter 180° verursacht werden.

Eine Ausführungsform sieht vor, dass das automatische Berechnen des zumindest einen Messparameters eine derartige Berechnung eines Rekonstruktionswinkelintervalls theta für die zumindest eine z-Position umfasst, dass das Rekonstruktionswinkelintervall theta für die zumindest eine z-Position direkt proportional zu dem atemkorrelierten Parameter $T_{cycle}$ ist. Das Rekonstruktionswinkelintervall theta ist insbesondere desto größer, je höher der atemkorrelierte Parameter $T_{cycle}$ ist bzw. je langsamer der Patient atmet. Das Rekonstruktionswinkelintervall theta hängt insbesondere von dem atemkorrelierten Parameter $T_{cycle}$ ab, wodurch das Rekonstruktionswinkelintervall theta patientenindividuell automatisch derart berechnet wird, dass insbesondere die Projektionsdaten an der zumindest einen z-Position den Atemzyklus des Patienten über die gesamte Zeitdauer des Atemzyklus abbilden.

Eine Ausführungsform sieht vor, dass das automatische Berechnen des zumindest einen Messparameters eine derartige Berechnung des Rekonstruktionswinkelintervalls theta für die zumindest eine z-Position umfasst, dass das Rekonstruktionswinkelintervall theta für die zumindest eine z-Position indirekt proportional zu der minimalen Röhrenrotationumlaufzeit $T_{rot}$ des Computertomographen bei der bildgebenden Untersuchung ist. Das Rekonstruktionswinkelintervall theta ist insbesondere desto kleiner, je größer die minimale Röhrenrotationumlaufzeit $T_{rot}$ des Computertomographen bei der bildgebenden Untersuchung ist. Das Rekonstruktionswinkelintervall theta ist insbesondere desto kleiner, je geringer die Röhren-Detektor-Geschwindigkeit ist. Das Rekonstruktionswinkelintervall theta hängt insbesondere von der minimalen Röhrenrotationumlaufzeit $T_{rot}$ des Computertomographen bei der bildgebenden Untersuchung ab, so dass das Rekonstruktionswinkelintervall theta gemäß der technischen Ausführung des Computertomographen automatisch derart berechnet wird, dass insbesondere die Projektionsdaten an der zumindest einen z-Position den Atemzyklus des Patienten über die gesamte Zeitdauer des Atemzyklus abbilden.

Eine Ausführungsform sieht vor, dass das automatische Berechnen des zumindest einen Messparameters umfasst, dass ein Produkt aus dem Rekonstruktionswinkelintervall theta für die zumindest eine z-Position und aus dem schicht-effektiven Röhrenstrom-Zeit-Produkt $I_{rot}$ des Computertomographen bei der bildgebenden Untersuchung konstant gehalten wird. Wenn beispielsweise das Rekonstruktionswinkelintervall theta mit einem Faktor multipliziert wird, wird automatisch das schicht-effektive Röhrenstrom-Zeit-Produkt $I_{rot}$ des Computertomographen bei der bildgebenden Untersuchung mit einem Kehrbruch des Faktors multipliziert. Dadurch wird insbesondere die Bildqualität BQ des Schichtbildes patientenübergreifend konstant gehalten. Wenn Schichtbilder an je unterschiedlichen z-Positionen rekonstruiert werden, weisen die Schichtbilder vorzugsweise die gleiche Bildqualität BQ auf.

Eine Ausführungsform sieht vor, dass die Projektionsdaten in zumindest einem Winkel relativ zu dem Patienten erfasst werden und die Projektionsdaten mit dem zumindest einen Winkel innerhalb eines Rekonstruktionswinkelintervalls theta für die zumindest eine z-Position ausgewählt werden, von welchen ein Schichtbild an der zumindest einen z-Position rekonstruiert wird. Die Projektionsdaten weisen insbesondere die aktuelle z-Position der Patientenliege bei der bildgebenden Untersuchung und insbesondere den zumindest einen Winkel des zumindest einen Röntgenstrahlers und des zumindest einen Röntgendetektors während der bildgebenden Untersuchung auf. Vorzugsweise werden für die Rekonstruktion des Schichtbildes an der zumindest einen z-Position die Projektionsdaten in dem zumindest einen Winkel innerhalb des Rekonstruktionswinkelintervalls theta verwendet.

Wenn mehrere Schichtbilder rekonstruiert werden, werden insbesondere für die Rekonstruktion eines einzelnen Schichtbildes vorzugsweise die Projektionsdaten in dem zumindest einen Winkel des jeweiligen Rekonstruktionswinkelintervalls theta verwendet. In anderen Worten umfasst das Rekonstruktionswinkelintervall theta mindestens 180° für jedes rekonstruierte Schichtbild.

Wenn die Projektionsdaten an der zumindest einen z-Position über die gesamte Zeitdauer des Atemzyklus erfasst werden, ist es besonders vorteilhaft nicht nur das Schichtbild, sondern zeitaufgelöste Schichtbilder aus den Projektionsdaten an der zumindest einen z-Position zu rekonstruieren. Der Atemzyklus des Patienten kann üblicherweise in den zeitaufgelösten Schichtbildern einfacher und besser abgebildet werden als in dem Schichtbild allein. Eine Zeitauflösung der zeitaufgelösten Schichtbilder wird insbesondere durch die minimale Röhrenrotationsumlaufzeit $T_{rot}$ des Computertomographen bei der bildgebenden Untersuchung limitiert bzw. vorgegeben. Für die Rekonstruktion eines zeitaufgelösten Schichtbildes der zeitaufgelösten Schichtbilder umfasst das Rekonstruktionswinkelintervall theta vorzugsweise den Winkelbereich von mindestens 180°.

Die Zeitauflösung definiert insbesondere eine maximale zeitliche Kohärenz von durch die Atembewegung bewegten Strukturen, insbesondere einer Hautoberfläche, eines Organs, eines Karzinoms und/oder eines Tumors, des Patienten. Wenn die Zeitauflösung N Sekunden umfasst, werden vorzugsweise innerhalb von N Sekunden die Projektionsdaten an der zumindest einen z-Position gemäß dem Rekonstruktionswinkelintervall theta erfasst. Die maximale zeitliche Kohärenz kann von anderen Arten an Bewegung des Patienten abhängen. Die maximale zeitliche Kohärenz bei der bildgebenden Untersuchung kann insbesondere optimiert sein, wenn keine Bewegung, insbesondere der durch die Atembewegung bewegten Strukturen, vorliegt. In anderen Worten, je weniger Bewegung des Patienten vorliegt, umso besser ist die maximale zeitliche Kohärenz.

Eine Ausführungsform sieht vor, dass das Rekonstruktionswinkelintervall theta für die zumindest eine z-Position direkt proportional zu einem Produkt aus einer relativen Zeitauflösung $\Delta T_{rel}$ und dem atemkorrelierten Parameter $T_{cycle}$ ist.

Die Zeitauflösung der zeitaufgelösten Schichtbilder wird vorzugsweise an die Atembewegung des Patienten angepasst, in dem eine relative Zeitauflösung $\Delta T_{rel}$ definiert wird. Die relative Zeitauflösung $\Delta T_{rel}$ wird insbesondere als Prozentsatz, vorzugsweise 5%, des atemkorrelierten Parameter $T_{cycle}$ definiert. In diesem Fall werden von den Projektionsdaten an der zumindest einen z-Position 20 zeitaufgelöste Schichtbilder rekonstruiert, welche die gesamte Zeitdauer des Atemzyklus abbilden. Die relative Zeitauflösung $\Delta T_{rel}$ kann der zweiten Parametergruppe zugeordnet werden, weil üblicherweise die relative Zeitauflösung $\Delta T_{rel}$, insbesondere durch den Nutzer, modifiziert werden kann.

Wenn das Rekonstruktionswinkelintervall theta durch die automatische Berechnung kleiner als 180° ist, wird vorzugsweise die relative Zeitauflösung $\Delta T_{rel}$ automatisch angepasst, insbesondere erhöht, so dass das Rekonstruktionswinkelintervall theta mindestens 180° beträgt. Üblicherweise verbleiben dabei der Röhrenstrom $I_{tube}$ und die minimale Röhrenrotationumlaufzeit $T_{rot}$ unverändert. In anderen Worten werden alle Patienten vorzugsweise derart gemessen, dass das Rekonstruktionswinkelintervall theta mindestens 180° beträgt.

Eine Ausführungsform sieht vor, dass das automatische Berechnen des zumindest einen Messparameters

- den atemkorrelierten Parameter $T_{cycle}$,
- den Röhrenstrom $I_{tube}$ des Computertomographen bei der bildgebenden Untersuchung,
- das schicht-effektives Röhrenstrom-Zeit-Produkt $I_{rot}$ des Computertomographen bei der bildgebenden Untersuchung,
- die minimale Röhrenrotationsumlaufzeit $T_{rot}$ des Computertomographen bei der bildgebenden Untersuchung,
- die relative Zeitauflösung $\Delta T_{rel}$ und
- das Rekonstruktionswinkelintervall theta für die zumindest eine z-Position berücksichtigt. Gemäß dieser Ausführung kann der zumindest eine Messparameter patientenindividuell, insbesondere unter Berücksichtigung der Atembewegung des Patienten, berechnet und festgelegt werden.

Eine Ausführungsform sieht vor, dass die bildgebende Untersuchung des Patienten in dem Messbereich mittels des Computertomographen gemäß

- dem atemkorrelierten Parameter $T_{cycle}$,
- den Röhrenstrom $I_{tube}$ des Computertomographen bei der bildgebenden Untersuchung,
- dem schicht-effektiven Röhrenstrom-Zeit-Produkt $I_{rot}$ des Computertomographen bei der bildgebenden Untersuchung,
- der minimalen Röhrenrotationsumlaufzeit $T_{rot}$ des Computertomographen bei der bildgebenden Untersuchung,
- der relativen Zeitauflösung $\Delta T_{rel}$ und
- dem Rekonstruktionswinkelintervall theta für die zumindest eine z-Position durchgeführt wird. Gemäß dieser Ausführung wird insbesondere die bildgebende Untersuchung des Patienten patientenindividuell, insbesondere unter Berücksichtigung der Atembewegung des Patienten, durchgeführt.

Eine Ausführungsform sieht vor, dass bei dem Durchführen der bildgebenden Untersuchung zusätzlich die Atembewegung des Patienten in Echtzeit erfasst wird und die Rekonstruktion des Schichtbildes an der zumindest einen z-Position aus den Projektionsdaten gemäß einer Atemphase der Atembewegung des Patienten erfolgt. Insbesondere wenn an der zumindest einen z-Position zeitaufgelöste Schichtbilder rekonstruiert werden, kann die Atemphase wenigstens einem der zeitaufgelösten Schichtbilder zugeordnet werden. Das wenigstens eine der zeitaufgelösten Schichtbilder zeigt insbesondere die z-Position gemäß der Atemphase. Beispielsweise kann auf dem Monitor durch den Nutzer die anzuzeigende Atemphase ausgewählt werden und die Planungseinheit zeigt insbesondere das wenigstens eine der zeitaufgelösten Schichtbilder an.

Wenn der Messbereich ein 3D-Volumen mit Ausdehnung in z-Richtung des Computertomographen aufweist und der Messbereich beispielsweise einen Thorax des Patienten umfasst, kann insbesondere der Thorax des Patienten gemäß der ausgewählten Atemphase des Patienten auf dem Monitor dargestellt werden. Beispielsweise kann gemäß der Auswahl durch den Nutzer der Patient einmal beim Einatmen und ein anderes Mal beim Ausatmen gezeigt werden.

Das Durchführen der bildgebenden Untersuchung des Patienten gemäß dem zumindest einen Messparameter in dem Messbereich mittels des Computertomographen kann insbesondere das Rekonstruieren von atemkorrelierten 4D-Bildern bzw. atemkorrelierten 3D-Bilder-Serien umfassen.

Der erfindungsgemäße Computertomograph umfasst

- eine Planungseinheit,
- eine Recheneinheit und
- eine Messeinheit, welche zumindest einen Röntgenstrahler und zumindest einen Röntgendetektor aufweist, wobei der Computertomograph dazu ausgebildet ist, ein erfindungsgemäßes Verfahren auszuführen.

Ein Computerprogrammprodukt, welches direkt in einen Speicher der programmierbaren Recheneinheit ladbar ist, weist Programmcode-Mittel auf, um ein erfindungsgemäßes Verfahren auszuführen, wenn das Computerprogrammprodukt in der Recheneinheit ausgeführt wird.

Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor der Recheneinheit geladen werden kann, der beispielsweise als Teil des Computertomographen ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software vgl. oben, gespeichert ist. Wenn diese Steuerinformationen Software von dem Datenträger gelesen und in der Recheneinheit und/oder Planungseinheit und/oder Messeinheit des Computertomographen gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Im Folgenden wird die Erfindung anhand der in den Figuren dargestellten Ausführungsbeispiele näher beschrieben und erläutert.

Es zeigen:

Fig. 1　einen erfindungsgemäßen Computertomographen,

Fig. 2　ein Flussdiagramm des erfindungsgemäßen Verfahrens,

Fig. 3 ein Ausführungsbeispiel des erfindungsgemäßen Verfahrens und

Fig. 4 eine Tabelle mit exemplarischen Messparametern, die gemäß einem Ausführungsbeispiel des erfindungsgemäßen Verfahrens berechnet sind.

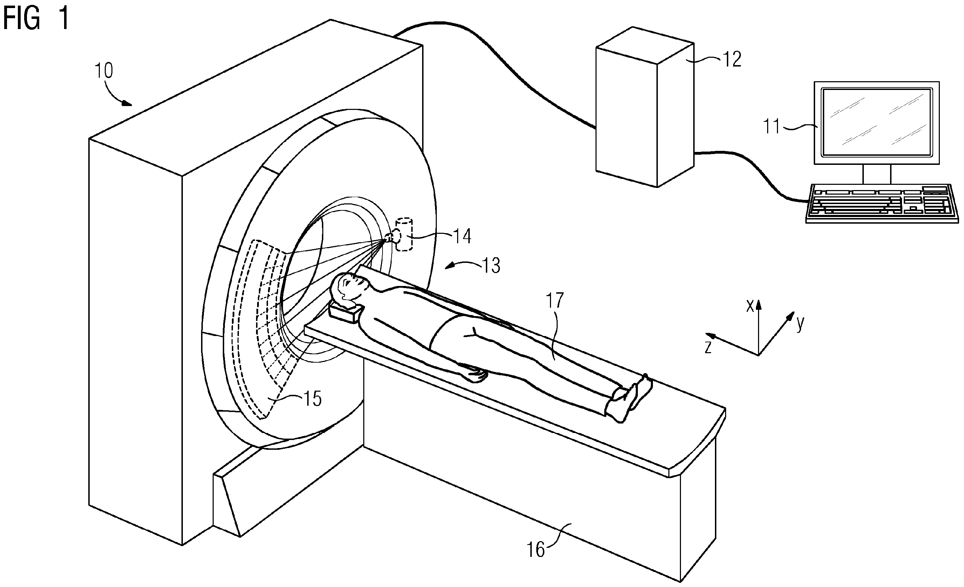

Fig. 1 zeigt einen erfindungsgemäßen Computertomographen 10, welcher eine Planungseinheit 11, eine Recheneinheit 12 und eine Messeinheit 13 umfasst. Die Messeinheit 13 umfasst zumindest einen Röntgenstrahler 14 und zumindest einen Röntgendetektor 15. Auf einer Patientenliege 16 ist der Patient 17 angeordnet.

Die Planungseinheit 11 kann den Monitor mit einer graphischen Benutzeroberfläche und ein Eingabegerät aufweisen. Üblicherweise kann ein Nutzer mit der Planungseinheit 11, insbesondere durch das Eingabegerät interagieren. Beispielsweise können dem Nutzer auf dem Monitor Computertomograph-Projektionsdaten und/oder ein Schichtbild angezeigt werden. Vorzugsweise kann der Nutzer auf dem Monitor eine Atemphase des Patienten auswählen und gemäß der Atemphase wird dem Nutzer das jeweilige Schichtbild angezeigt.

Der Computertomograph 10 ist mit der Planungseinheit 11 und der Recheneinheit 12 zu einem Datenaustausch verbunden.

Der Computertomograph 10 kann vorzugsweise den zumindest einen Röntgenstrahler 14 und den zumindest einen Röntgendetektor 15, welche relativ zueinander in einem festen Abstand angeordnet sind, um die Patientenliege 16, insbesondere den Patienten 17, rotieren. Der Computertomograph 10 erfasst bei einem Durchführen der bildgebenden Untersuchung, in welchem Winkel sich der zumindest eine Röntgenstrahler 14 und der zumindest eine Röntgendetektor 15 relativ zu der Patientenliege 17 befinden. Vorzugsweise ordnet der Computertomograph 10 Projektionsdaten, die in dem Winkel des zumindest einen Röntgenstrahlers 14 und des zumindest einen Röntgendetektors 15 relativ zu der Patientenliegen 16 eine Beschreibung des Winkels zu, wodurch bei der Rekonstruktion des Schichtbildes von den Projektionsdaten der Winkel berücksichtigt werden kann.

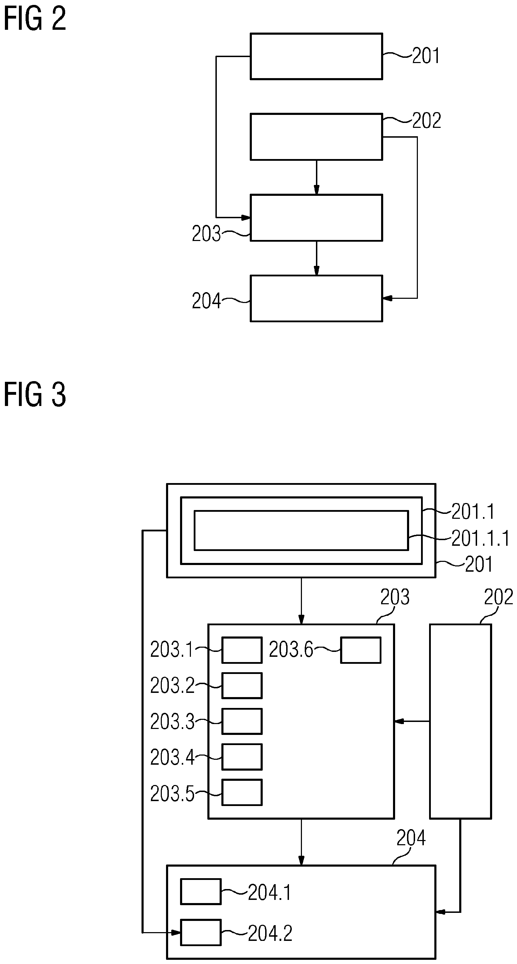

Fig. 2 zeigt ein Flussdiagramm des erfindungsgemäßen Verfahrens zum Durchführen der bildgebenden Untersuchung des Patienten 17 mittels des Computertomographen 10. Das erfindungsgemäße Verfahren umfasst die Verfahrensschritte 201-204.

Verfahrensschritt 201 kennzeichnet das Erfassen einer Atembewegung des Patienten 17, wobei von der Atembewegung des Patienten 17 ein atemkorrelierter Parameter $T_{cycle}$ ermittelt wird, welcher eine Zeitdauer eines Atemzyklus der Atembewegung beschreibt.

Verfahrensschritt 202 kennzeichnet das Festlegen eines Messbereichs der bildgebenden Untersuchung, wobei der Messbereich zumindest eine z-Position aufweist.

Verfahrensschritt 203 kennzeichnet das automatisches Berechnen zumindest eines Messparameters gemäß der Atembewegung, wobei der atemkorrelierte Parameter $T_{cycle}$ als Eingangsparameter für das automatische Berechnen des zumindest einen Messparameters derart verwendet wird, dass bei einem Durchführen der bildgebenden Untersuchung gemäß dem zumindest einen Messparameter Projektionsdaten an der zumindest einen z-Position über die gesamte Zeitdauer des Atemzyklus erfasst werden können.

In den Verfahrensschritt 203 gehen der atemkorrelierte Parameter $T_{cycle}$ und die zumindest eine z-Position ein.

Verfahrensschritt 204 kennzeichnet Durchführen der bildgebenden Untersuchung des Patienten gemäß dem zumindest einen Messparameter in dem Messbereich mittels des Computertomographen, wobei die Projektionsdaten akquiriert werden, welche an der zumindest einen z-Position den Atemzyklus des Patienten 17 über die gesamte Zeitdauer des Atemzyklus abbilden.

In den Verfahrensschritt 204 gehen der Messbereich und der zumindest eine automatisch berechnete Messparameter ein.

Fig. 3 zeigt ein Ausführungsbeispiel des erfindungsgemäßen Verfahrens.

Die nachfolgende Beschreibung beschränkt sich im Wesentlichen auf die Unterschiede zu dem Ausführungsbeispiel in Fig. 2, wobei bezüglich gleich bleibender Verfahrensschritte auf die Beschreibung des Ausführungsbeispiels in Fig. 2 verwiesen wird. Im Wesentlichen gleich bleibende Verfahrensschritte sind grundsätzlich mit den gleichen Bezugszeichen beziffert.

Verfahrensschritt 201.1 kennzeichnet, dass der atemkorrelierte Parameter $T_{cycle}$ derart ermittelt wird, dass die Atembewegung in periodische Segmente unterteilt wird und die Zeitdauer des Atemzyklus gemäß einem Median von Zeitdauern der periodischen Segmente berechnet wird.

Verfahrensschritt 201.1.1 kennzeichnet, dass der atemkorrelierte Parameter $T_{cycle}$ gemäß einer Standardabweichung von den Zeitdauern der periodischen Segmente angepasst wird.

Verfahrensschritt 203.1 kennzeichnet, dass das automatische Berechnen des zumindest einen Messparameters eine derartige Berechnung eines Röhrenstroms $I_{tube}$ des Computertomographen 10 bei der bildgebenden Untersuchung umfasst, dass der Röhrenstrom $I_{tube}$ indirekt proportional zum atemkorrelierten Parameter $T_{cycle}$ ist.

Verfahrensschritt 203.2 kennzeichnet, dass das automatische Berechnen des zumindest einen Messparameters eine derartige Berechnung eines schicht-effektiven Röhrenstrom-Zeit-Produkts $I_{rot}$ umfasst, dass das schicht-effektive Röhrenstrom-Zeit-Produkt $I_{rot}$ einem Produkt aus dem Röhrenstrom $I_{tube}$ des Computertomographen bei der bildgebenden Untersuchung und aus einer minimalen Röhrenrotationumlaufzeit $T_{rot}$ des Computertomographen 10 bei der bildgebenden Untersuchung entspricht.

Verfahrensschritt 203.3 kennzeichnet, dass das automatische Berechnen des zumindest einen Messparameters eine derartige Berechnung des Rekonstruktionswinkelintervalls theta für die zumindest eine z-Position umfasst, dass das Rekonstruktionswinkelintervall theta für die zumindest eine z-Position direkt proportional zu dem atemkorrelierten Parameter $T_{cycle}$ ist.

Verfahrensschritt 203.4 kennzeichnet, dass das Rekonstruktionswinkelintervall theta für die zumindest eine z-Position direkt proportional zu einem Produkt aus einer relativen Zeitauflösung $\Delta T_{rel}$ und dem atemkorrelierten Parameter $T_{cycle}$ ist.

Verfahrensschritt 203.5 kennzeichnet, dass das automatische Berechnen des zumindest einen Messparameters eine derartige Berechnung des Rekonstruktionswinkelintervalls theta für die zumindest eine z-Position umfasst, dass das Rekonstruktionswinkelintervall theta für die zumindest eine z-Position indirekt proportional zu der minimalen Röhrenrotationumlaufzeit $T_{rot}$ des Computertomographen 10 bei der bildgebenden Untersuchung ist.

Verfahrensschritt 203.6 kennzeichnet, dass das automatische Berechnen des zumindest einen Messparameters umfasst, dass ein Produkt aus dem Rekonstruktionswinkelintervall theta für die zumindest eine z-Position und aus dem schicht-effektiven Röhrenstrom-Zeit-Produkt $I_{rot}$ des Computertomographen 10 bei der bildgebenden Untersuchung konstant gehalten wird.

Aus diesem Vorgehen folgt, dass das automatische Berechnen des zumindest einen Messparameters

- den atemkorrelierten Parameter $T_{cycle}$,
- den Röhrenstrom $I_{tube}$ des Computertomographen 10 bei der bildgebenden Untersuchung,
- das schicht-effektive Röhrenstrom-Zeit-Produkt $I_{rot}$ des Computertomographen 10 bei der bildgebenden Untersuchung,
- die minimale Röhrenrotationsumlaufzeit $T_{rot}$ des Computertomographen 10 bei der bildgebenden Untersuchung,
- die relative Zeitauflösung $\Delta T_{rel}$ und
- das Rekonstruktionswinkelintervall theta für die zumindest eine z-Position berücksichtigt.

Verfahrensschritt 204.1 kennzeichnet, dass die Projektionsdaten in zumindest einem Winkel relativ zu dem Patienten 17 erfasst werden und die Projektionsdaten mit dem zumindest einen Winkel innerhalb des Rekonstruktionswinkelintervalls theta für die zumindest eine z-Position ausgewählt werden, von welchen ein Schichtbild an der zumindest einen z-Position rekonstruiert wird.

Aus diesem Vorgehen folgt, dass die bildgebende Untersuchung des Patienten 17 in dem Messbereich mittels des Computertomographen 10 gemäß

- dem atemkorrelierten Parameter $T_{cycle}$,
- dem Röhrenstrom $I_{tube}$ des Computertomographen 10 bei der bildgebenden Untersuchung,
- dem schicht-effektiven Röhrenstrom-Zeit-Produkt $I_{rot}$ des Computertomographen 10 bei der bildgebenden Untersuchung,
- der minimalen Röhrenrotationsumlaufzeit $T_{rot}$ des Computertomographen 10 bei der bildgebenden Untersuchung,
- der relativen Zeitauflösung $\Delta T_{rel}$ und
- dem Rekonstruktionswinkelintervall theta für die zumindest eine z-Position durchgeführt wird.

Verfahrensschritt 204.2 kennzeichnet, dass bei dem Durchführen der bildgebenden Untersuchung zusätzlich die Atembewegung des Patienten 17 in Echtzeit erfasst wird und eine Rekonstruktion des Schichtbildes an der zumindest einen z-Position aus den Projektionsdaten gemäß einer Atemphase der Atembewegung des Patienten 17 erfolgt.

In den Verfahrensschritt 204.2 geht das Erfassen der Atembewegung gemäß Verfahrensschritt 201 ein.

Bei diesem Vorgehen wurden die Verfahrensschritte 201.1, 201.1.1, 203.1, 203.2, 203.3, 203.4, 203.5, 203.6, 204.1 und 204.2 gemeinsam berücksichtigt. Selbstverständlich ist es denkbar, diese Verfahrensschritte auch einzeln durchzuführen bzw. beliebig zu kombinieren.

Eine erste Formel zur Berechnung des abhängigen Parameters $I_{tube}$ lautet:

$$I_{tube} = \frac{I_{ges}}{T_{cycle}}$$

Der Röhrenstrom $I_{tube}$ hat die Einheit [mA]. Die Gesamtdosis $I_{ges}$ hat die Einheit [mAs]. Der atemkorrelierte Parameter $T_{cyc-le}$ hat die Einheit [s].

Eine zweite Formel zur Berechnung des abhängigen Parameters $I_{rot}$ lautet:

$$I_{rot} = I_{tube} \cdot T_{rot}$$

Das schicht-effektive Röhrenstrom-Zeit-Produkt $I_{rot}$ hat die Einheit [mAs].

Eine dritte Formel zur Berechnung des abhängigen Parameters theta lautet:

$$theta = \frac{\Delta T_{rel} \cdot T_{cycle} \cdot 360°}{T_{rot}}$$

Das Rekonstruktionswinkelintervall theta hat die Einheit [°].

Eine vierte Formel zur Berechnung der Bildqualität BQ lautet:

$$BQ = theta \cdot I_{rot} = const$$

Der atemkorrelierte Parameter $T_{cycle}$ kann in einen Parameter Atemzyklen-pro-Minute ApM umgerechnet werden.

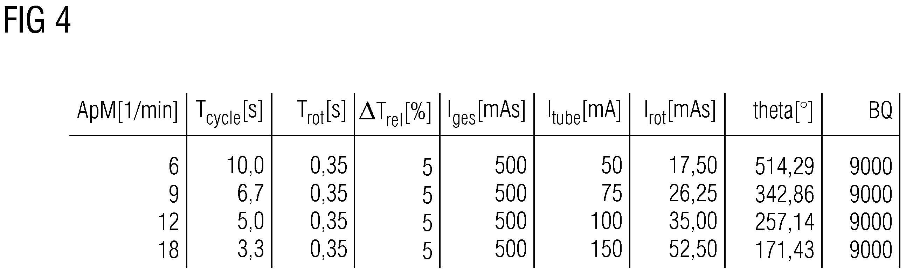

Fig. 4 eine Tabelle mit exemplarischen Messparametern, die gemäß einer Ausführungsform des erfindungsgemäßen Verfahrens, insbesondere gemäß der ersten, zweiten, dritten und vierten Formel berechnet sind.

Die nachfolgende Beschreibung beschränkt sich im Wesentlichen auf die Unterschiede zu dem Ausführungsbeispiel in Fig. 2 und Fig. 3, wobei bezüglich gleich bleibender Verfahrensschritte auf die Beschreibung des Ausführungsbeispiels in Fig. 2 und in Fig. 3 verwiesen wird. Im Wesentlichen gleich bleibende Verfahrensschritte sind grundsätzlich mit den gleichen Bezugszeichen beziffert.

Die dargestellten Werte dienen lediglich nur zur Veranschaulichung des hier gezeigten Ausführungsbeispiels.

Fig. 4 zeigt, dass der Röhrenstrom $I_{tube}$ indirekt proportional zum atemkorrelierten Parameter $T_{cycle}$ ist.

Fig. 4 zeigt, dass das schicht-effektive Röhrenstrom-Zeit-Produkt $I_{rot}$ einem Produkt aus dem Röhrenstrom $I_{tube}$ des Computertomographen 10 bei der bildgebenden Untersuchung und aus der minimalen Röhrenrotationumlaufzeit $T_{rot}$ des Computertomographen 10 bei der bildgebenden Untersuchung entspricht.

Fig. 4 zeigt, dass das Rekonstruktionswinkelintervall theta für die zumindest eine z-Position direkt proportional zu einem Produkt aus der relativen Zeitauflösung $\Delta T_{rel}$ und dem atemkorrelierten Parameter $T_{cycle}$ ist.

Fig. 4 zeigt, dass das Rekonstruktionswinkelintervall theta für die zumindest eine z-Position direkt proportional zu dem atemkorrelierten Parameter $T_{cycle}$ ist.

Fig. 4 zeigt, dass ein Produkt BQ aus dem Rekonstruktionswinkelintervall theta für die zumindest eine z-Position und aus dem schicht-effektiven Röhrenstrom-Zeit-Produkt $I_{rot}$ des Computertomographen bei der bildgebenden Untersuchung konstant ist.

Obwohl die Erfindung im Detail durch das bevorzugte Ausführungsbeispiel näher illustriert und beschrieben wurde, ist die Erfindung nicht durch die offenbarten Beispiele eingeschränkt. Variationen hiervon können vom Fachmann abgeleitet werden, ohne den Schutzumfang der Erfindung, wie er durch die nachfolgenden Patentansprüche definiert wird, zu verlassen.

## Patentansprüche

1. Verfahren zum Durchführen einer bildgebenden Untersuchung eines Patienten mittels eines Computertomographen, umfassend die Schritte:

- Erfassen einer Atembewegung des Patienten, wobei von der Atembewegung des Patienten ein atemkorrelierter Parameter $T_{cycle}$ ermittelt wird, welcher eine Zeitdauer eines Atemzyklus der Atembewegung beschreibt,
- Festlegen eines Messbereichs der bildgebenden Untersuchung, wobei der Messbereich zumindest eine z-Position aufweist,
- automatisches Berechnen zumindest eines Messparameters gemäß der Atembewegung, wobei der atemkorrelierte Parameter $T_{cycle}$ als Eingangsparameter für das automatische Berechnen des zumindest einen Messparameters derart verwendet wird, dass bei einem Durchführen der bildgebenden Untersuchung gemäß dem zumindest einen Messparameter Projektionsdaten an der zumindest einen z-Position über die gesamte Zeitdauer des Atemzyklus erfasst werden, und
- Durchführen der bildgebenden Untersuchung des Patienten gemäß dem zumindest einen Messparameter in dem Messbereich mittels des Computertomographen, wobei die Projektionsdaten akquiriert werden, welche an der zumindest einen z-Position den Atemzyklus des Patienten über die gesamte Zeitdauer des Atemzyklus abbilden,

wobei das automatische Berechnen des zumindest einen Messparameters eine derartige Berechnung eines Röhrenstroms $I_{tube}$ des Computertomographen bei der bildgebenden Untersuchung umfasst, dass der Röhrenstrom $I_{tube}$ indirekt proportional zum atemkorrelierten Parameter $T_{cycle}$ ist, wobei eine Gesamtdosis $I_{ges}$ gemäß einem Produkt aus dem Röhrenstrom $I_{tube}$ mit dem atemkorrelierten Parameter $T_{cycle}$ konstant gehalten wird.

2. Verfahren nach Anspruch 1, wobei der atemkorrelierte Parameter $T_{cycle}$ derart ermittelt wird, dass die Atembewegung in periodische Segmente unterteilt wird und die Zeitdauer des Atemzyklus gemäß einem Median von Zeitdauern der periodischen Segmente berechnet wird.

3. Verfahren nach Anspruch 2, wobei der atemkorrelierte Parameter $T_{cycle}$ gemäß einer Standardabweichung von den Zeitdauern der periodischen Segmente angepasst wird.

4. Verfahren nach einem der vorhergehenden Ansprüche, wobei das automatische Berechnen des zumindest einen Messparameters eine derartige Berechnung eines schicht-effektiven Röhrenstrom-Zeit-Produkts $I_{rot}$ umfasst, dass das schicht-effektive Röhrenstrom-Zeit-Produkt $I_{rot}$ einem Produkt aus einem Röhrenstrom $I_{tube}$ des Computertomographen bei der bildgebenden Untersuchung und aus einer

minimalen Röhrenrotationumlaufzeit $T_{rot}$ des Computertomographen bei der bildgebenden Untersuchung entspricht.

5. Verfahren nach einem der vorhergehenden Ansprüche, wobei das automatische Berechnen des zumindest einen Messparameters eine derartige Berechnung eines Rekonstruktionswinkelintervalls theta für die zumindest eine z-Position umfasst, dass das Rekonstruktionswinkelintervall theta für die zumindest eine z-Position direkt proportional zu dem atemkorrelierten Parameter $T_{cycle}$ ist.

6. Verfahren nach Anspruch 5, wobei das Rekonstruktionswinkelintervall theta für die zumindest eine z-Position direkt proportional zu einem Produkt aus einer relativen Zeitauflösung $\Delta T_{rel}$ und dem atemkorrelierten Parameter $T_{cycle}$ ist.

7. Verfahren nach einem der vorhergehenden Ansprüche, wobei das automatische Berechnen des zumindest einen Messparameters eine derartige Berechnung eines Rekonstruktionswinkelintervalls theta für die zumindest eine z-Position umfasst, dass das Rekonstruktionswinkelintervall theta für die zumindest eine z-Position indirekt proportional zu einer minimalen Röhrenrotationumlaufzeit $T_{rot}$ des Computertomographen bei der bildgebenden Untersuchung ist.

8. Verfahren nach einem der vorhergehenden Ansprüche, wobei das automatische Berechnen des zumindest einen Messparameters umfasst, dass ein Produkt aus einem Rekonstruktionswinkelintervall theta für die zumindest eine z-Position und aus einem schicht-effektiven Röhrenstrom-Zeit-Produkt $I_{rot}$ des Computertomographen bei der bildgebenden Untersuchung konstant gehalten wird.

9. Verfahren nach einem der vorhergehenden Ansprüche, wobei die Projektionsdaten in zumindest einem Winkel relativ zu dem Patienten erfasst werden und die Projektionsdaten mit dem zumindest einen Winkel innerhalb eines Rekonstruktionswinkelintervalls theta für die zumindest eine z-Position ausgewählt werden, von welchen ein Schichtbild an der zumindest einen z-Position rekonstruiert wird.

10. Verfahren nach einem der vorhergehenden Ansprüche, wobei das automatische Berechnen des zumindest einen Messparameters

- den atemkorrelierten Parameter $T_{cycle}$,
- einen Röhrenstrom $I_{tube}$ des Computertomographen bei der bildgebenden Untersuchung,
- ein schicht-effektives Röhrenstrom-Zeit-Produkt $I_{rot}$ des Computertomographen bei der bildgebenden Untersuchung,

- eine minimale Röhrenrotationsumlaufzeit $T_{rot}$ des Computertomographen bei der bildgebenden Untersuchung,
- eine relative Zeitauflösung $\Delta T_{rel}$ und
- ein Rekonstruktionswinkelintervall theta für die zumindest eine z-Position berücksichtigt.

11. Verfahren nach einem der vorhergehenden Ansprüche, wobei die bildgebende Untersuchung des Patienten in dem Messbereich mittels des Computertomographen gemäß

- dem atemkorrelierten Parameter $T_{cycle}$,
- einem Röhrenstrom $I_{tube}$ des Computertomographen bei der bildgebenden Untersuchung,
- einem schicht-effektiven Röhrenstrom-Zeit-Produkt $I_{rot}$ des Computertomographen bei der bildgebenden Untersuchung,
- einer minimalen Röhrenrotationsumlaufzeit $T_{rot}$ des Computertomographen bei der bildgebenden Untersuchung,
- einer relativen Zeitauflösung $\Delta T_{rel}$ und
- einem Rekonstruktionswinkelintervall theta für die zumindest eine z-Position durchgeführt wird.

12. Verfahren nach einem der vorhergehenden Ansprüche, wobei bei dem Durchführen der bildgebenden Untersuchung zusätzlich die Atembewegung des Patienten in Echtzeit erfasst wird und eine Rekonstruktion eines Schichtbildes an der zumindest einen z-Position aus den Projektionsdaten gemäß einer Atemphase der Atembewegung des Patienten erfolgt.

13. Computertomograph, umfassend

- eine Planungseinheit,
- eine Recheneinheit und
- eine Messeinheit, welche zumindest einen Röntgenstrahler und zumindest einen Röntgendetektor aufweist,

wobei der Computertomograph dazu ausgebildet ist, ein Verfahren nach einem der vorhergehenden Ansprüche auszuführen.

14. Computerprogrammprodukt, welches direkt in einen Speicher einer programmierbaren Recheneinheit ladbar ist, mit Programmcode-Mitteln, um ein Verfahren nach einem der Ansprüche 1 bis 12 auszuführen, wenn das Computerprogrammprodukt in der Recheneinheit des Computertomographen nach Anspruch 13 ausgeführt wird.

**Claims**

1. Method for performing an imaging examination of a patient by means of a computer tomograph, comprising the steps:

- capturing a respiratory movement of the patient, wherein a respiration-correlated parameter $T_{cycle}$ which describes a time duration of a respiratory cycle of the respiratory movement is determined from the respiratory movement of the patient,
- specifying a measurement region of the imaging examination, wherein the measurement region has at least one z-position,
- automatically calculating at least one measurement parameter in accordance with the respiratory movement, wherein the respiration-correlated parameter $T_{cycle}$ is used as an input parameter for the automatic calculation of the at least one measurement parameter, such that when the imaging examination is performed in accordance with the at least one measurement parameter, projection data at the at least one z-position can be captured over the complete time duration of the respiratory cycle, and
- performing the imaging examination of the patient in accordance with the at least one measurement parameter in the measurement region by means of the computer tomograph, wherein the projection data is acquired which depicts the respiratory cycle of the patient at the at least one z-position over the complete time duration of the respiratory cycle,

wherein the automatic calculation of the at least one measurement parameter comprises calculating a tube current $I_{tube}$ of the computer tomograph during the imaging examination in such a way that the tube current $I_{tube}$ is indirectly proportional to the respiration-correlated parameter $T_{cycle}$, wherein an overall dose $I_{ges}$ according to a product from the tube current $I_{tube}$ is kept constant with the respiration-correlated parameter $T_{cycle}$.

2. Method according to claim 1, wherein the respiration-correlated parameter $T_{cycle}$ is determined by dividing the respiratory movement into periodic segments and calculating the time duration of the respiratory cycle in accordance with a median of time durations of the periodic segments.

3. Method according to claim 2, wherein the respiration-correlated parameter $T_{cycle}$ is adapted according to a standard deviation of the time durations of the periodic segments.

4. Method according to one of the preceding claims, wherein the automatic calculation of the at least one measurement parameter comprises calculating a slice-effective tube current-time product $I_{rot}$ in such a way that the slice-effective tube current-time product $I_{rot}$ corresponds to a product of a tube current $I_{tube}$ of the computer tomograph during the imaging examination and a minimum tube rotation time $T_{rot}$ of the computer tomograph during the imaging examination.

5. Method according to one of the preceding claims, wherein the automatic calculation of the at least one measurement parameter comprises calculating a reconstruction angular interval theta for the at least one z-position in such a way that the reconstruction angular interval theta for the at least one z-position is directly proportional to the respiration-correlated parameter $T_{cycle}$.

6. Method according to claim 5, wherein the reconstruction angular interval theta for the at least one z-position is directly proportional to a product of a relative temporal resolution $\Delta T_{rel}$ and the respiration-correlated parameter $T_{cycle}$.

7. Method according to one of the preceding claims, wherein the automatic calculation of the at least one measurement parameter comprises calculating a reconstruction angular interval theta for the at least one z-position in such a way that the reconstruction angular interval theta for the at least one z-position is indirectly proportional to a minimum tube rotation time $T_{rot}$ of the computer tomograph during the imaging examination.

8. Method according to one of the preceding claims, wherein the automatic calculation of the at least one measurement parameter comprises holding constant a product of a reconstruction angular interval theta for the at least one z-position and a slice-effective tube current-time product $I_{rot}$ of the computer tomograph during the imaging examination.

9. Method according to one of the preceding claims, wherein the projection data is captured in at least one angle relative to the patient and the projection data with the at least one angle within a reconstruction angular interval theta for the at least one z-position is selected as a basis for reconstructing a slice image at the at least one z-position.

10. Method according to one of the preceding claims, wherein the automatic calculation of the at least one measurement parameter takes into account

- the respiration-correlated parameter $T_{cycle}$,
- a tube current $I_{tube}$ of the computer tomograph during the imaging examination,
- a slice-effective tube current-time product $I_{rot}$

of the computer tomograph during the imaging examination,
- a minimum tube rotation time $T_{rot}$ of the computer tomograph during the imaging examination,
- a relative temporal resolution $\Delta T_{rel}$, and
- a reconstruction angular interval theta for the at least one z-position.

11. Method according to one of the preceding claims, wherein the imaging examination of the patient in the measurement region by means of the computer tomograph is performed in accordance with

- the respiration-correlated parameter $T_{cycle}$,
- a tube current $I_{tube}$ of the computer tomograph during the imaging examination,
- a slice-effective tube current-time product $I_{rot}$ of the computer tomograph during the imaging examination,
- a minimum tube rotation time $T_{rot}$ of the computer tomograph during the imaging examination,
- a relative temporal resolution $\Delta T_{rel}$, and
- a reconstruction angular interval theta for the at least one z-position.

12. Method according to one of the preceding claims, wherein during the performance of the imaging examination, the respiratory movement of the patient is additionally captured in real time and a reconstruction of a slice image at the at least one z-position from the projection data is effected in accordance with a respiratory phase of the respiratory movement of the patient.

13. Computer tomograph comprising

- a planning unit,
- an arithmetic unit, and
- a measuring unit having at least one x-ray source and at least one x-ray detector,

wherein the computer tomograph is designed to execute a method according to one of the preceding claims.

14. Computer program product which can be loaded directly into a memory of a programmable arithmetic unit, having program code means for executing a method according to one of claims 1 to 12 when the computer program product is executed in the arithmetic unit of the computed tomograph according to claim 13.

**Revendications**

1. Procédé pour effectuer un examen d'un patient par imagerie au moyen d'un tomodensitomètre assisté par ordinateur, comprenant les stades :

- détection d'un mouvement respiratoire du patient, dans lequel on détermine du mouvement respiratoire du patient un paramètre $T_{cycle}$ corrélé à la respiration, qui décrit une durée d'un cycle respiratoire du mouvement respiratoire,
- fixation d'une partie de mesure de l'examen par imagerie, la partie de mesure ayant au moins une position z,
- calcul automatique d'au moins un paramètre de mesure suivant le mouvement respiratoire, le paramètre $T_{cycle}$ corrélé à la respiration étant utilisé comme paramètre d'entrée pour le calcul automatique du au moins un paramètre de mesure de manière à ce que, lorsque l'on effectue l'examen par imagerie suivant le au moins un paramètre de mesure, des données de projection sur la au moins une position z sont détectées sur toute la durée du cycle respiratoire, et
- exécution de l'examen du patient par imagerie suivant le au moins un paramètre de mesure dans la partie de mesure au moyen du tomodensitomètre assisté par ordinateur, dans lequel on acquiert les données de projection, qui reproduisent à la au moins une position z le cycle respiratoire du patient sur toute la durée du cycle respiratoire,

dans lequel le calcul automatique du au moins un paramètre de mesure comprend un calcul d'un courant $I_{tube}$ de tube du tomodensitomètre assisté par ordinateur lors de l'examen par imagerie de manière à ce que le courant $I_{tube}$ de tube soit inversement proportionnel au paramètre $T_{cycle}$ corrélé à la respiration, dans lequel on maintient constante une dose $I_{ges}$ d'ensemble suivant un produit du courant $I_{tube}$ de tube par le paramètre $T_{cycle}$ corrélé à la respiration.

2. Procédé suivant la revendication 1, dans lequel on détermine le paramètre $T_{cycle}$ corrélé à la respiration de manière à subdiviser le mouvement respiratoire en segments périodiques et à calculer la durée du cycle respiratoire suivant une médiane des durées des segments périodiques.

3. Procédé suivant la revendication 2, dans lequel on adapte l'écart-type paramètre $T_{cycle}$ corrélé à la respiration suivant un écart type des durées des segments périodiques.

4. Procédé suivant l'une des revendications précédentes, dans lequel le calcul automatique du au moins un paramètre de mesure comprend un calcul tel d'un produit $I_{rot}$ efficace de tranche courant de tube-temps que le produit $I_{rot}$ efficace de tranche courant de tube-temps corresponde à un produit d'un courant $I_{tube}$ de tube du tomodensitomètre assisté par ordinateur lors de l'examen par imagerie et d'un temps $T_{rot}$ minimum de tour de rotation du tube du tomodensitomètre assisté par ordinateur lors de l'examen par imagerie.

5. Procédé suivant l'une des revendications précédentes, dans lequel le calcul automatique du au moins un paramètre de mesure comprend un calcul tel d'un intervalle theta angulaire de reconstruction pour la au moins une position z que l'intervalle theta angulaire de reconstruction pour la au moins une position z soit directement proportionnel au paramètre $T_{cycle}$ corrélé à la respiration.

6. Procédé suivant la revendication 5, dans lequel l'intervalle theta angulaire de reconstruction pour la au moins une position z est directement proportionnel à un produit d'une résolution $\Delta T_{re1}$ temporelle relative par le paramètre $T_{cycle}$ corrélé à la respiration.

7. Procédé suivant l'une des revendications précédentes, dans lequel le calcul automatique du au moins un paramètre de mesure comprend un calcul tel d'un intervalle theta angulaire de reconstruction pour la au moins une position z que l'intervalle theta angulaire de reconstruction pour la au moins une position z soit inversement proportionnelle à un temps $T_{rot}$ minimum de tour de rotation de tube du tomodensitomètre assisté par ordinateur lors de l'examen par imagerie.

8. Procédé suivant l'une des revendications précédentes, dans lequel le calcul automatique du au moins un paramètre de mesure comprend qu'un produit d'un intervalle theta angulaire de reconstruction pour la au moins une position z par un produit $I_{rot}$ efficace de tranche courant de tube-temps du tomodensitomètre assisté par ordinateur soit maintenu constant lors de l'examen par imagerie.

9. Procédé suivant l'une des revendications précédentes, dans lequel on détecte les données de projection dans au moins un angle par rapport au patient et on choisit les données de projection ayant le au moins un angle dans un intervalle theta angulaire de reconstruction pour la au moins une position z, par lesquelles on reconstruit une tomographie à la au moins une position z.

10. Procédé suivant l'une des revendications précédentes, dans lequel le calcul automatique du au moins un paramètre de mesure prend en compte

- le paramètre $T_{cycle}$ corrélé à la respiration,
- un courant $I_{tube}$ de tube du tomodensitomètre assisté par ordinateur lors de l'examen par imagerie,
- un produit $I_{rot}$ efficace de tranche courant de tube-temps du tomodensitomètre assisté par ordinateur lors de l'examen par imagerie,
- un temps $T_{rot}$ minimum de tour de rotation de tube du tomodensitomètre assisté par ordinateur lors de l'examen par imagerie,
- une résolution $\Delta T_{re1}$ temporelle relative et
- un intervalle theta angulaire de reconstruction pour la au moins une position z.

11. Procédé suivant l'une des revendications précédentes, dans lequel on effectue l'examen du patient par imagerie dans la partie de mesure au moyen du tomodensitomètre assisté par ordinateur suivant

- le paramètre $T_{cycle}$ corrélé à la respiration,
- un courant $I_{tube}$ de tube du tomodensitomètre assisté par ordinateur lors de l'examen par imagerie,
- un produit $I_{rot}$ efficace de tranche courant de tube -temps du tomodensitomètre assisté par ordinateur lors de l'examen par imagerie,
- un temps $T_{rot}$ minimum de tour de rotation de tube du tomodensitomètre assisté par ordinateur lors de l'examen par imagerie,
- une résolution $\Delta T_{re1}$ temporelle relative et
- un intervalle theta angulaire de reconstruction pour la au moins une position z.

12. Procédé suivant l'une des revendications précédentes, dans lequel lors de l'exécution de l'examen par imagerie, on détecte en outre en temps réel le mouvement respiratoire du patient et il se produit une reconstruction d'une tomographie à la au moins une position z à partir des données de projection suivant une phase respiratoire du mouvement respiratoire du patient.

13. Tomodensitomètre assisté par ordinateur comprenant

- une unité de planification,
- une unité de calcul et
- une unité de mesure qui a au moins une source de rayons X et au moins un détecteur de rayons X, dans lequel le tomodensitomètre assisté par ordinateur est constitué pour effectuer un procédé suivant l'une des revendications précédentes.

14. Produit de programme d'ordinateur, qui peut être chargé directement dans une mémoire d'une unité informatique programmable, comprenant des moyens de code de programme pour effectuer un procédé suivant l'une des revendications 1 à 12 lorsque le produit de programme d'ordinateur est réalisé dans l'unité informatique du tomodensitomètre assisté par ordinateur suivant la revendication 13.

FIG 1

EP 3 363 363 B1

FIG 2

FIG 3

## FIG 4

| ApM[1/min] | $T_{cycle}$[s] | $T_{rot}$[s] | $\Delta T_{rel}$[%] | $I_{ges}$[mAs] | $I_{tube}$[mA] | $I_{rot}$[mAs] | theta[°] | BQ |
|---|---|---|---|---|---|---|---|---|
| 6 | 10,0 | 0,35 | 5 | 500 | 50 | 17,50 | 514,29 | 9000 |
| 9 | 6,7 | 0,35 | 5 | 500 | 75 | 26,25 | 342,86 | 9000 |
| 12 | 5,0 | 0,35 | 5 | 500 | 100 | 35,00 | 257,14 | 9000 |
| 18 | 3,3 | 0,35 | 5 | 500 | 150 | 52,50 | 171,43 | 9000 |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110286574 A1
- EP 0370341 A2